# EUROPEAN PATENT SPECIFICATION

(11) **EP 3 522 785 B1**
(45) Date of publication and mention of the grant of the patent: **07.12.2022**
(21) Application number: 17791179.9
(22) Date of filing: 03.10.2017
(51) Int. Cl.: A61B 6/04, A61B 6/00, A61B 5/053, A61B 5/103, A61B 5/107, A61B 5/00, A61B 5/0507

(54) **MAMMOGRAPHY-APPARATUS OF MEASURING A CONTACT AREA BETWEEN A BREAST AND THE MAMMOGRAPHY-APPARATUS**
MAMMOGRAFIEVORRICHTUNG ZUR MESSUNG DER KONTAKTFLÄCHE ZWISCHEN EINER BRUST UND DER MAMMOGRAFIEVORRICHTUNG
APPAREIL DE MAMMOGRAPHIE DE MESURE D'UNE ZONE DE CONTACT ENTRE UN SEIN ET L'APPAREIL DE MAMMOGRAPHIE

(30) Priority: 05.10.2016 NL 2017578
(43) Date of publication of application: 14.08.2019
(73) Proprietor: SIGMASCREENING B.V., 7521 AN Enschede (NL)
(72) Inventor: DEN HEETEN, Gerardus, Johannes, 1105 AZ Amsterdam (NL); GRIMBERGEN, Cornelis, Antonius, 1105 AZ Amsterdam (NL)
(74) Representative: Van Breda, Jacobus
(86) International application number: PCT/NL2017/050653
(87) International publication number: WO 2018/067005

(56) References cited:
- US-A- 5 999 836
- US-A1- 2012 020 455
- US-A1- 2014 341 338
- US-A1- 2015 265 186

## Description

The invention relates to a mammography-apparatus for detecting malignant cells in a breast, which apparatus comprises an x-ray source and an x-ray detector that cooperates with the x-ray source for providing an x-ray image of said breast, and further comprises a paddle for flattening the breast by pressing it against said x-ray detector.

Such a mammography-apparatus is known from US2008/0240346 in which a pressure detector is applied for detecting pressures applied by a compression plate to an image capturing base, and wherein the pressure detector may comprise a pressure sensitive sheet.

US2012/0020455 teaches a mammography-apparatus for detecting malignant cells in a breast comprising an x-ray source and an x-ray detector that cooperates with the x-ray source for providing an x-ray image of said breast, and further comprising a paddle for flattening the breast by pressing it against an x-ray detector cover, and comprising contact area measuring sensors for measuring a contact area between the breast and the mammography-apparatus, wherein the sensors are mounted in the detector surface or at the underside of the paddle.

In US2007/0121782 the size of a breast is determined according to the size of the compression paddle, or by pre-exposing the breast with a small amount of radiation by the breast size detection means prior to imaging. A solid-state detector which is used for the pre-exposure will have different pixel values between the breast area and the area directly exposed by the radiation. Alternatively a CCD camera may be used, or a strain gauge provided in the object table, or temperature sensors provided on the side of the object table facing the chest.

In US2008/0043904 a compression plate comprising elastically deformable elements is used, wherein an adaptation of the shape of the individual compression plate elements is achieved to the anatomy of the female breast.

A mammography-apparatus according to the preamble is further known from WO2011/102713 and comprises a contact area measuring device employing an optical system for measuring a contact area between the breast and the paddle.

According to WO2011/102713 the contact area measuring device can be used in a dual fashion. In an embodiment in which the force that is applied to the breast is measured, this force together with the contact area provides an estimation of the average pressure that is applied to the breast. This average pressure can then be controlled at a pre-established level so as to avoid unnecessary and avoidable pain during imaging.

Further, the measured contact area between the breast and the paddle resulting from the breast-compression can be used together with the pre-established force-level, to calculate and apply a specific mean compression pressure independent of the dimensions of the individual breast. Knowing and controlling this specific mean compression pressure leads to a better standardization of the mammography operation, with improved accuracy of screening whilst avoiding unnecessary pain for the persons being screened.

US 2014/341338 which corresponds to EP 2 819 581 teaches that the contact area measuring device can be embodied with at least a first laminate of an electrically insulating material and an electrically low resistance material, which first laminate is provided on a side of the paddle facing the breast, wherein the electrically low resistance material is sandwiched between the paddle and the insulating material. This opens the way to carry out an electrical measurement of the actual area at which the breast and paddle are in contact with each other. According to US 2014/341338 (and EP 2 819 581) the measuring of the contact area between the breast and the paddle of the mammography-apparatus that further comprises an x-ray detector with a cover, is then carried out such that the electrically conducting x-ray detector cover and the electrically low resistance material of the first laminate are connected to a measurement circuit which is powered by an alternating current excitation source, wherein the electrical potential is measured between the x-ray detector and the electrically low resistance material of the first laminate on the paddle, and that said electrical potential is used as a measure for the contact area between the breast and the paddle. This measurement of the contact area is then based on the change of capacitance between the x-ray detector cover and the electrically low resistance material of the first laminate on the paddle, as determined by the breast placed between the paddle and the x-ray detector cover.

US 2014/341338 which corresponds to EP 2 819 581 further discloses that it may be beneficial that the contact area measuring device comprises in addition to the first laminate on the paddle which therefore always is present, a second laminate of an electrically low resistance material sandwiched between two layers of an electrically insulating material, which second laminate is provided on a side of the x-ray detector cover facing the breast with one of the layers of insulating material provided against the x-ray detector cover. In this manner both the upper and lower contact areas with the paddle and the x-ray detector cover, respectively can be measured. According to US 2014/341338 (and EP 2 819 581) this is however not a requirement, one can do also with only the first laminate provided on the paddle.

Relatively high costs are incurred with the apparatus according to US 2014/341338 (and EP 2 819 581) due to the fact that the known mammography-apparatus is embodied with interchangeable paddles that each have to be provided with a laminate to make the contact area measurement between the breast and the apparatus possible. The instant invention is aimed at reducing the costs of the apparatus according to US 2014/341338 (and EP 2 819 581) without sacrificing the benefits that it provides in terms of reliable and accurate measurement of the contact area between the mammography-apparatus and the breast.

The invention is embodied in the mammography-apparatus of measuring the contact area between the breast and the mammography-apparatus as specified in the appended claims.

In a first aspect of the invention the contact area measuring device is only provided on or incorporated in the detector cover facing the breast and the paddle is or paddles are embodied without a contact area measuring device. Within the scope of this invention the term "detector cover" embraces and includes an electrical insulation layer which is provided immediately on the detector and which separates the contact area measuring device from the bare detector. It is therefore not necessarily a part of the device which during use is in contact with the breast.

The invention is based on breaking with the prejudice that the contact area measurement should always at least be carried out regarding the contact area between the breast and the paddle. The inventors have however found that this is not necessary and that by taking appropriate measures it is also possible to only detect the contact area between the breast and the detector cover, without sacrificing the reliability and accuracy of the measurement. The invention brings the advantage to save costs since the replaceable paddles no longer require the application of a laminate as taught by EP 2 819 581. On the contrary with the invention it is only required to apply a single suitable laminate on the detector cover or to incorporate such laminate in the detector cover.

Suitably the contact area measuring device comprises a laminate of an electrically low resistance material sandwiched between two layers of an electrically insulating material, which laminate is provided on a side of the x-ray detector cover facing the breast with one of the layers of insulating material provided against the x-ray detector cover. It is possible that the laminate for application on the x-ray detector cover comprises a layer of an electrically low resistance material and only one layer of an electrically insulating material, and that a separate electrically insulating material is applied between the laminate and the x-ray detector cover. Within the terms of this application both embodiments are considered to constitute a laminate of an electrically low resistance material sandwiched between two layers of an electrically insulating material.

The laminate might be provided on the side of the x-ray detector cover facing the breast. The measurement of the contact area between the breast and the x-ray detector cover is carried out by connecting the x-ray detector cover and the electrically low resistance material of the laminate on the x-ray detector cover to a measurement circuit which is powered by an alternating current excitation source, and that with reference to the potential of the x-ray detector cover the electrical potential of the electrically low resistance material of the laminate on the x-ray detector cover is measured, which electrical potential is then used as measure for the contact area between the breast and the x-ray detector cover.

The measurement of the contact area between the breast and the x-ray detector cover will then be based on the change of capacitance between the x-ray detector cover and the electrically low resistance material of the laminate on the x-ray detector cover, as this is determined by the breast placed between the paddle and the x-ray detector cover.

In all embodiments the laminate on the x-ray detector cover is preferably homogenous and substantially x-ray transparent.

The laminate on the x-ray detector cover preferably comprises materials with a low level of distortion or attenuation of the x-rays that are required for imaging the breast. On the other hand the insulating material of the laminate must suit the purpose of enabling the capacitance measurement according to the method of the invention. Suitably therefore both objectives can be met when the insulating material of the laminate is a thermoplastic having a relative dielectric constant of at least 1.8. Appropriately the insulating material of the laminate is then selected from the group comprising polyethylene, cyanoacrylate, polycarbonate. The dielectric constants of these details are 2 (polyethylene); 3 (cyanoacrylate); and 4.4 (polycarbonate). The laminate must be insulated from the conducting x-ray detector cover. For this purpose the laminate is preferably completed with an electrically insulating material on the side which will be provided to the detector cover. In this construction the electrically low resistance material of the laminate is sandwiched between two insulating layers.

Suitably the electrically low resistance material of the laminate is selected from the group comprising an electron-doped semiconductor and graphene.

Another preferable material to use for the electrically low resistance material of the laminate is silver nanowire. This silver nanowire preferably has a thickness of much less than 1 µm.

The invention will hereinafter be further elucidated with reference to the drawing providing a schematic figure of an embodiment of the mammography-apparatus according to the invention, which is not limiting to the appended claims.

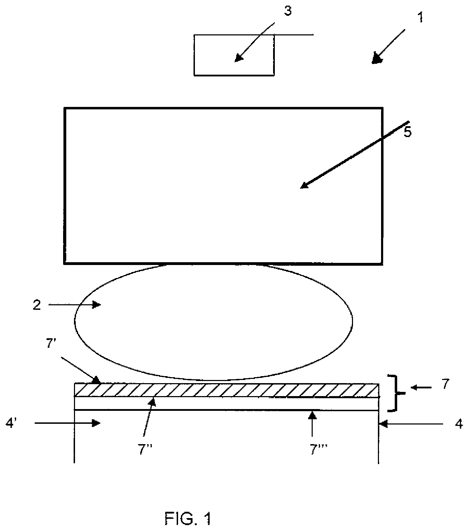

In the drawing figure 1 shows the mammography-apparatus of the invention, which is used for detecting malignant cells in a breast 2, wherein the x-ray detector is provided with a laminate.

The mammography-apparatus 1 comprises in known way an x-ray source 3 and an x-ray detector 4' that cooperates with the x-ray source 3 for providing an x-ray image of the breast 2. The apparatus further has a paddle 5 for flattening the breast 2 by pressing the breast against the x-ray detector cover 4.

According to the invention there is a contact area measuring device for measuring a contact area between the breast 2 and the detector cover 4, which is embodied with a laminate 7 of an electrically insulating material and an electrically low resistance material.

In the shown embodiment the contact area measuring device comprises a laminate 7 of an electrically low resistance material 7" sandwiched between two layers 7', 7‴ of electrically insulating materials, which laminate 7 is provided on a side of the x-ray detector cover 4 facing the breast 2. The insulating material 7' ensures isolation of the breast 2 from the electrically low resistance material 7" of the laminate 7. In the shown example the laminate 7 is provided on the x-ray detector cover 4 wherein the intermediate insulating material 7‴ attached to the x-ray detector cover 4 forms an integral part of the laminate 7. It is however also possible that this latter intermediate insulating material 7‴ is applied separately from the laminate 7.

Preferably the insulating material 7' of the laminate 7 is a thermoplastic having a relative dielectric constant of at least 1.8. The insulating material 7' of the laminate 7 is preferably selected from the group comprising polyethylene, cyanoacrylate, polycarbonate. Further the insulating material 7' of the laminate 7 has a thickness in the range 0.1-0.25 millimeters, preferably approximately 0.17 mm.

The electrically low resistance material 7" of the laminate 7 preferably has a specific resistivity of less than 5×10⁻⁶ Ohm.m, and is selected from the group comprising an electron-doped semiconductor and graphene. It is preferred however that the electrically low resistance material 7" is silver nanowire, having a thickness of much less than 1 µm.

The mammography-apparatus 1 that is shown in figure 1 is used as follows. The contact area between the breast 2 and the x-ray detector cover 4 is measured by connecting x-ray detector cover 4 and the electrically low resistance material 7" of the laminate 7 on the x-ray detector cover 4 to a measurement circuit which is powered by an alternating current excitation source. Then with reference to the potential of the x-ray detector cover 4, the electrical potential is measured of the electrically low resistance material 7" of the laminate 7 on the x-ray detector cover 4, and this electrical potential is used as a measure for the contact area between the breast 2 and the x-ray detector cover 4.

The way in which the measurement circuit can be implemented can be in the form of applying voltage dividers or by measuring the time it takes to fully charge the capacitance between the low electrical resistivity material 7" of the laminate 7 and the x-ray detector cover 4. The way this can be implemented is known to the person skilled in the art and requires no further elucidation with reference to the drawing.

It is explicitly remarked that the above elucidation of the features of the invention are not intended to limit the appended claims to the specific examples that are provided herewith. On the contrary, the invention is solely defined by the appended claims and the above elucidation merely serves to elucidate the claims if any ambiguity would reside in these claims. Without departing from the scope of the claims many variations are therefore feasible.

## Claims

1. Mammography-apparatus (1) for detecting malignant cells in a breast (2) comprising an x-ray source (3) and an x-ray detector (4') that cooperates with the x-ray source (3) for providing an x-ray image of said breast (2), and further comprising a paddle (5) for flattening the breast (2) by pressing it against an x-ray detector cover (4), and comprising a contact area measuring device for measuring a contact area between the breast (2) and the mammography-apparatus (1), wherein the contact area measuring device comprises a laminate (7) of an electrically low resistance material (7") sandwiched between two layers of an electrically insulating material (7', 7‴), with the contact area measuring device being embodied with a measurement circuit and equipped to be connected during use to an alternating current excitation source for measuring the electrical potential between the X-ray detector cover (4) and the electrically low resistance material (7") of the laminate (7) on the detector cover (4) **characterized in that** the paddle (5) is replaceable and embodied without a contact area measuring device and that the contact area measuring device is only provided on or incorporated in the detector cover (4) facing the breast (2).

2. Mammography-apparatus according to claim 1, **characterized in that** the laminate (7) is provided on a side of the x-ray detector cover (4) facing the breast (2) with one of the layers of insulating material (7', 7‴) provided against the x-ray detector cover (4).

3. Mammography-apparatus according to claim 1 or 2, **characterized in that** the laminate (7) is homogenous and substantially x-ray transparent.

4. Mammography-apparatus according to any one of the previous claims, **characterized in that** the insulating material (7') of the laminate (7) is a thermoplastic having a relative dielectric constant of at least 1.8.

5. Mammography-apparatus according to any one of claims 1-4, **characterized in that** the insulating material (7') of the laminate (7) is selected from the group comprising polyethylene, cyanoacrylate, polycarbonate.

6. Mammography-apparatus
according to any one of the previous claims, **characterized in that** the insulating material (7') of the laminate (7) has a thickness in the range 0.1-0.25 millimeters, preferably approximately 0.17 mm.

7. Mammography-apparatus according to any one of claims 1-6, **characterized in that** the electrically low resistance material (7") of the laminate (7) has a specific resistivity of less than 5×10⁻⁶ Ohm.m.

8. Mammography-apparatus according to any one of the previous claims, **characterized in that** the electrically low resistance material (7") of the laminate (7) is selected from the group comprising an electron-doped semiconductor and graphene.

9. Mammography-apparatus according to any one of the previous claims, **characterized in that** the electrically low resistance material (7") of the laminate (7) is silver nanowire.

10. Mammography-apparatus according to claim 9, **characterized in that** the silver nanowire has a thickness of less than 1 µm.

## Patentansprüche

1. Mammographievorrichtung (1) zum Erkennen von malignen Zellen in einer Brust (2), umfassend eine Röntgenquelle (3) und einen Röntgendetektor (4'), der mit der Röntgenquelle (3) zusammenwirkt, um ein Röntgenbild der Brust (2) zu erzeugen, und ferner umfassend ein Paddle (5) zum Abflachen der Brust (2), indem sie gegen eine Röntgendetektorabdeckung (4) gedrückt wird, und mit einer Kontaktflächenmessvorrichtung zum Messen einer Kontaktfläche zwischen der Brust (2) und der Mammographievorrichtung (1), wobei die Kontaktflächenmessvorrichtung ein Laminat (7) aus einem elektrisch niederohmigen Material (7") umfasst, das zwischen zwei Schichten aus einem elektrisch isolierenden Material (7', 7‴), wobei die Kontaktflächenmessvorrichtung mit einer Messschaltung ausgestattet ist und so eingerichtet ist, dass sie während des Gebrauchs an eine Wechselstromanregungsquelle angeschlossen werden kann, um das elektrische Potential zwischen der Röntgendetektorabdeckung (4) und dem elektrisch niederohmigen Material (7") des Laminats (7) auf der Detektorabdeckung (4) zu messen, **dadurch gekennzeichnet, dass** das Paddle (5) austauschbar und ohne eine Kontaktflächenmessvorrichtung ausgeführt ist und dass die Kontaktflächenmessvorrichtung nur auf der der Brust (2) zugewandten Detektorabdeckung (4) vorgesehen oder darin eingebaut ist.

2. Mammographievorrichtung nach Anspruch 1, **dadurch gekennzeichnet, dass** das Laminat (7) auf einer der Brust (2) zugewandten Seite der Röntgendetektorabdeckung (4) vorgesehen ist, wobei eine der Schichten aus isolierendem Material (7', 7‴) gegen die Röntgendetektorabdeckung (4) vorgesehen ist.

3. Mammographievorrichtung nach Anspruch 1 oder 2, **dadurch gekennzeichnet, dass** das Laminat (7) homogen und im Wesentlichen röntgentransparent ist.

4. Mammographievorrichtung nach einem der vorhergehenden Ansprüche, wobei das isolierende Material (7') des Laminats (7) ein thermoplastischer Kunststoff mit einer relativen Dielektrizitätskonstante von mindestens 1,8 ist.

5. Mammographievorrichtung nach einem der Ansprüche 1-4, **dadurch gekennzeichnet, dass** das isolierende Material (7') des Laminats (7) aus der Gruppe ausgewählt ist, die Polyethylen, Cyanoacrylat und Polycarbonat umfasst.

6. Mammographievorrichtung nach einem der vorhergehenden Ansprüche, **dadurch gekennzeichnet, dass** das isolierende Material (7') des Laminats (7) eine Dicke im Bereich von 0,1-0,25 Millimetern, vorzugsweise etwa 0,17 mm, aufweist.

7. Mammographievorrichtung nach einem der Ansprüche 1-6, **dadurch gekennzeichnet, dass** das elektrisch niederohmige Material (7") des Laminats (7) einen spezifischen Widerstand von weniger als 5×10⁻⁶ Ohm.m aufweist.

8. Mammographievorrichtung nach einem der vorhergehenden Ansprüche, wobei das elektrisch niederohmige Material (7") des Laminats (7) ausgewählt ist aus der Gruppe, die einen elektronendotierten Halbleiter und Graphen umfasst.

9. Mammographievorrichtung nach einem der vorhergehenden Ansprüche, **dadurch gekennzeichnet, dass** das elektrisch niederohmige Material (7") des Laminats (7) ein Silber-Nanodraht ist.

10. Mammographievorrichtung nach Anspruch 9, **dadurch gekennzeichnet, dass** der Silber-Nanodraht eine Dicke von weniger als 1 µm aufweist.

## Revendications

1. Appareil de mammographie (1) pour détecter des cellules malignes dans un sein (2) comprenant une source de rayons X (3) et un détecteur de rayons X (4') qui coopère avec la source de rayons X (3) pour fournir une image de rayons X dudit sein (2), et comprenant en outre une palette (5) pour aplatir le sein (2) en le pressant contre un couvercle de détecteur (4) de rayons X, et comprenant un dispositif de mesure de zone de contact pour mesurer une zone de contact entre le sein (2) et l'appareil de mammographie (1), dans lequel le dispositif de mesure de zone de contact comprend un stratifié (7) d'un matériau à faible résistance électrique (7") pris en sandwich entre deux couches d'un matériau électriquement isolant (7', 7‴), le dispositif de mesure de zone de contact étant conçu avec un circuit de mesure et étant équipé pour être connecté pendant l'utilisation à une source d'excitation à courant alternatif pour mesurer le potentiel électrique entre le couvercle de détecteur (4) de rayons X et le matériau à faible résistance électrique (7") du stratifié (7) sur le couvercle de détecteur (4), **caractérisé en ce que** la palette (5) est remplaçable et conçue sans dispositif de mesure de zone de contact, et **en ce que** le dispositif de mesure de zone de contact est uniquement prévu sur, ou incorporé dans, le couvercle de détecteur (4) faisant face au sein (2).

2. Appareil de mammographie selon la revendication 1, **caractérisé en ce que** le stratifié (7) est prévu sur un côté du couvercle de détecteur (4) de rayons X faisant face au sein (2), l'une des couches du matériau isolant (7', 7‴) étant prévue contre le couvercle de détecteur (4) de rayons X.

3. Appareil de mammographie selon la revendication 1 ou 2, **caractérisé en ce que** le stratifié (7) est homogène et sensiblement transparent aux rayons X.

4. Appareil de mammographie selon l'une quelconque des revendications précédentes, **caractérisé en ce que** le matériau isolant (7') du stratifié (7) est un thermoplastique ayant une constante diélectrique relative d'au moins 1,8.

5. Appareil de mammographie selon l'une quelconque des revendications 1 à 4, **caractérisé en ce que** le matériau isolant (7') du stratifié (7) est choisi dans le groupe comprenant le polyéthylène, le cyanoacrylate et le polycarbonate.

6. Appareil de mammographie selon l'une quelconque des revendications précédentes, **caractérisé en ce que** le matériau isolant (7') du stratifié (7) a une épaisseur dans la plage de 0,1 à 0,25 millimètre, de préférence environ 0,17 mm.

7. Appareil de mammographie selon l'une quelconque des revendications 1 à 6, **caractérisé en ce que** le matériau à faible résistance électrique (7") du stratifié (7) a une résistivité spécifique inférieure à 5×10⁻⁶ Ohm.m.

8. Appareil de mammographie selon l'une quelconque des revendications précédentes, **caractérisé en ce que** le matériau à faible résistance électrique (7") du stratifié (7) est choisi dans le groupe comprenant un semi-conducteur dopé aux électrons et le graphène.

9. Appareil de mammographie selon l'une quelconque des revendications précédentes, **caractérisé en ce que** le matériau à faible résistance électrique (7") du stratifié (7) est un nanofil d'argent.

10. Appareil de mammographie selon la revendication 9, **caractérisé en ce que** le nanofil d'argent a une épaisseur inférieure à 1 µm.
